# EUROPÄISCHE PATENTSCHRIFT

(11) **EP 1 439 901 B1**
(45) Veröffentlichungstag und Bekanntmachung des Hinweises auf die Patenterteilung: **20.06.2007**
(21) Anmeldenummer: 02767428.2
(22) Anmeldetag: 26.08.2002
(51) Int. Cl.: B01J 8/04, B01J 8/06, C07C 51/265

(54) **REAKTORANORDNUNG FÜR DIE DURCHFÜHRUNG KATALYTISCHER GASPHASENREAKTIONEN, INSBESONDERE ZUR GEWINNUNG VON PHTHALSÄUREANHYDRID**
REACTOR ARRANGEMENT FOR CARRYING OUT CATALYTIC GAS PHASE REACTIONS, ESPECIALLY TO OBTAIN PHTHALIC ACID ANHYDRIDE
ENSEMBLE REACTEUR DESTINE A LA MISE EN OEUVRE DE REACTIONS CATALYTIQUES EN PHASE GAZEUSE, EN PARTICULIER A LA PREPARATION D'ANHYDRIDE PHTALIQUE

(30) Priorität: 12.09.2001 DE 10144857
(43) Veröffentlichungstag der Anmeldung: 28.07.2004
(73) Patentinhaber: MAN DWE GmbH, 94469 Deggendorf (DE)
(72) Erfinder: GÜTLHUBER, Friedrich, 85579 Neubiberg (DE)
(74) Vertreter: Paustian, Othmar
(86) Internationale Anmeldenummer: PCT/EP2002/009515
(87) Internationale Veröffentlichungsnummer: WO 2003/022418

(56) Entgegenhaltungen:
- EP-A- 0 686 633
- DE-A- 19 742 821
- DE-A- 19 807 018
- US-A- 3 871 445
- US-A- 5 161 605
- US-A- 5 986 146
- PATENT ABSTRACTS OF JAPAN vol. 014, no. 227 (C-0718), 15. Mai 1990 (1990-05-15) & JP 02 056238 A (JGC CORP;OTHERS: 01), 26. Februar 1990 (1990-02-26)

## Beschreibung

Die Erfindung betrifft eine Reaktoranordnung gemäß Gattungsbegriff des Patentanspruchs 1.

Aus DE-1 210 791 ist es bekannt, die stark exotherme Herstellung von Phthalsäureanhydrid (PSA) durch katalytische Luftoxidation von Naphthalin unter Aufteilung der Wärmetönung in mehreren hintereinandergeschalteten, immer größer werdenden Reaktionskammern mit dazwischen erfolgender Kühlung durch Wassereinspritzung gesamtheitlich unterhalb der Explosionsgrenze durchzuführen.

Des weiteren ist es aus DE 25 46 268 C3 bekannt, PSA durch katalytische Luftoxidation von o-Xylol oder Naphthalin in einem salzbadgekühlten Röhrenreaktor mit zwei aufeinanderfolgenden Katalysatorschichten herzustellen, um die, vor allem bei verhältnismäßig hoher Beladung der Luft mit dem zu oxidierenden Prozeßgas, zunächst entstehenden Nebenprodukte, wie vor allem Phthalid bzw. Naphtochinon, abzubauen. In diesem Fall soll die Beladung bis in den Explosionsbereich hinein erfolgen. Die Reaktionsrohre sollen bis zu 3,5 m lang sein.

Des weiteren gibt US-A-4,215,053 für die PSA-Synthese ein zweistufiges Oxidationsverfahren an, wobei der zweite Schritt unter schärferen Bedingungen erfolgen soll als der erste und bei längerer Verweilzeit.

Nach EP 0 453 951 B1 erfolgt die PSA-Synthese in einem Röhrenreaktor mit zwei aufeinanderfolgenden Reaktionszonen und getrennter Salzbadkühlung beider Zonen gemäß DE-A-2 201 528.

Gemäß EP 0 686 633 A1 finden zur PSA-Synthese zwei aufeinanderfolgende salzbadgekühlte Röhrenreaktoren mit zusätzlicher Zwischen- und Nachkühlung vor bzw. nach dem zweiten Reaktor Verwendung, wobei die Rohre des zweiten Reaktors wesentlich länger sein sollen als diejenigen des ersten. Dem aus dem ersten Reaktor austretenden Reaktionsprodukt wird vor Eintritt in den zweiten Reaktor nochmals Einsatzmaterial zugesetzt. Dabei wird der zweite Reaktor näher an der Entflammbarkeitsgrenze betrieben als der erste.

DE-197 42 821 A1 zeigt zur PSA-Synthese einen einem Hauptreaktor nachgeschalteten Festbettreaktor, der mit einem vorausgehenden und nachfolgenden Kühler in einem mit dem Hauptreaktor über eine Rohrleitung verbundenen Gehäuse zusammengefaßt ist.

Aus DE 198 07 018 A1 sind für die PSA-Synthese zwei aufeinanderfolgende Reaktoren bekannt, von denen der erste ein salzbadgekühlter Röhrenreaktor und der zweite, sogenannte Nachreaktor ein ungekühlter Festbettreaktor ist. Hier soll mit sehr hohen Beladungen gefahren werden. Zweck des Nachreaktors ist es, die Produktqualität und Ausbeute zu steigern sowie die Lebensdauer des Katalysators zu verbessern.

DE 198 52 894 A1 zeigt schließlich noch zur PSA-Synthese eine Folge von Katalysatorfestbetten mit dazwischenliegenden Kühleinheiten in einem einzigen Gehäuse.

Aus US-A-5 986 146 ist eine gattungsgemäße Reaktoranordnung mit mehreren in Reihe hintereinander geschalteten Katalysatorfestbetten bekannt. Zwischen die Katalysatorfestbetten ist jeweils ein Wärmetauscher geschaltet, die als mehrere einzelne Wärmetauscher oder als ein einteiliger Wärmetauscher ausgebildet sein können. Gemäß einer Ausführungsform wird ein erster Teil der Reaktionsgaskomponenten dem ersten Katalysatorfestbett zugeleitet und dann weiter durch die anschließenden Katalysatorfestbetten und die jeweils zwischengeschalteten Wärmetauscher geführt. Wenigstens ein zweiter Teil der Reaktionsgaskomponenten wird einem der weiteren Katalysatorfestbetten direkt zugeleitet. Hierdurch soll die Konzentration der auf diese Weise zugeführten Reaktionsgaskomponenten im Reaktionsgasgemisch gesteuert werden, um einerseits einen optimalen Reaktionsverlauf zu erzielen und andererseits die Konzentration dieser Reäktionsgaskomponenten in dem Reaktionsgasgemisch unterhalb der Explosionsgrenze zu halten. Nachteilig hierbei ist, dass die Verringerung des Explosionsrisikos aufgrund der erforderlichen Zuführung der Reaktionsgaskomponenten in mehreren Reaktionsgasströmen längs des Hauptreaktors technisch sehr aufwendig und damit kostenintensiv ist. Zudem kann das Explosionsrisiko nur bei mindestens zwei hintereinander geschalteten Katalysatorfestbetten verringert werden, da ansonsten eine Aufteilung des Reaktionsgasstromes nicht möglich ist. Darüber hinaus ist bei der bekannten Reaktoranordnung das dem Reaktionsgasgemisch zur Verfügung stehende Volumen relativ groß, so dass im Fall einer Explosion auch die Explosionsauswirkungen entsprechend groß sind, was entsprechend schwere Schäden an der Anlage nach sich zieht.

Wie bereits in der vorausgehend erörterten Schrift DE 197 42 821 A1 zum Ausdruck kommt, verringert sich das bei hoher Beladung auftretende Explosionsrisiko mit den in den diversen Apparaten und Rohrleitungen eingeschlossenen Volumina. Erfindungsgemäß wird nun angestrebt, mit besonders hoher Beladung und entsprechend hohem Wirkungsgrad zu fahren, wobei man zwangsläufig weit in den Explosionsbereich hineingerät.

Die Aufgabe der Erfindung liegt daher darin, eine gattungsgemäße Reaktoranordnung so zu verbessern, dass mit geringem Aufwand bei einer höheren Beladung das Explosionsrisiko und mögliche Explosionsschäden beträchtlich verringert werden.

Diese Aufgabe ist erfindungsgemäß durch die Kennzeichnungsmerkmale des Anspruchs 1 gelöst. Die Unteransprüche geben davon ausgehend vorteilhafte Ausgestaltungsmöglichkeiten an.

Dadurch, daß der Nachreaktor mit vorausgehender Kühlstufe, die von einem Röhrenkühler gebildet wird, mit dem Hauptreaktor, der ein flüssigkeitsgekühlter Röhrenreaktor ist, in ein-und demselben Gehäuse angeordnet ist, lassen sich die Gasvolumina am Austritt des Hauptreaktors außerordentlich klein halten. Dazu noch erfordern Nachreaktor und Kühlstufe kein eigenes Gehäuse, ebenso wie dazwischenliegende Rohrleitungen entfallen. Nachdem der Nachreaktor in der Regel wiederum ein selbst ungekühlter Festbettreaktor sein wird, kann der betreffende Gehäuseteil leicht abnehmbar an den Mantelteil des Hauptreaktors angeflanscht sein, wodurch sich der Austausch des Katalysators von Hauptreaktor wie Nachreaktor einfach gestaltet. Dessen ungeachtet erscheint unter Verwendung der Erfindung eine Katalysatorlebensdauer von mehr als 5 Jahren bei einer Ausbeute von über 100 g/nm³ Phthalsäureanhydrid realisierbar.

Nachfolgend werden einige Ausführungsbeispiele der Erfindung anhand der begleitenden Zeichnungen genauer beschrieben. Von diesen zeigt:
Fig. 1 einen schematischen Längsschnitt durch eine erfindungsgemäße Reaktoranordhung samt angeschlossenem Kühlsystem,
Fig. 2 ein Detail aus einer ähnlichen Reaktoranordnung, wobei jedoch der Nachreaktor einen geringeren Durchmesser als der Hauptreaktor besitzt,
Fig. 3 ein ähnliches Detail, bei dem eine Querschnittsverringerung des Nachreaktors auf andere Weise bewirkt ist,
Fig. 4 ein Detail mit einem Nachreaktor anderer Gestalt,
Fig. 5 ein Detail vom Anschluß einer Kühlstufe nach Fig. 1 an den Hauptreaktor in etwas abgewandelter Ausführung,
Fig. 6 ein Detail vom Anschluß einer Kühlstufe anderer Bauart und
Fig. 7 einen schematischen Längsschnitt durch eine Kühlstufe wieder anderer Bauart.

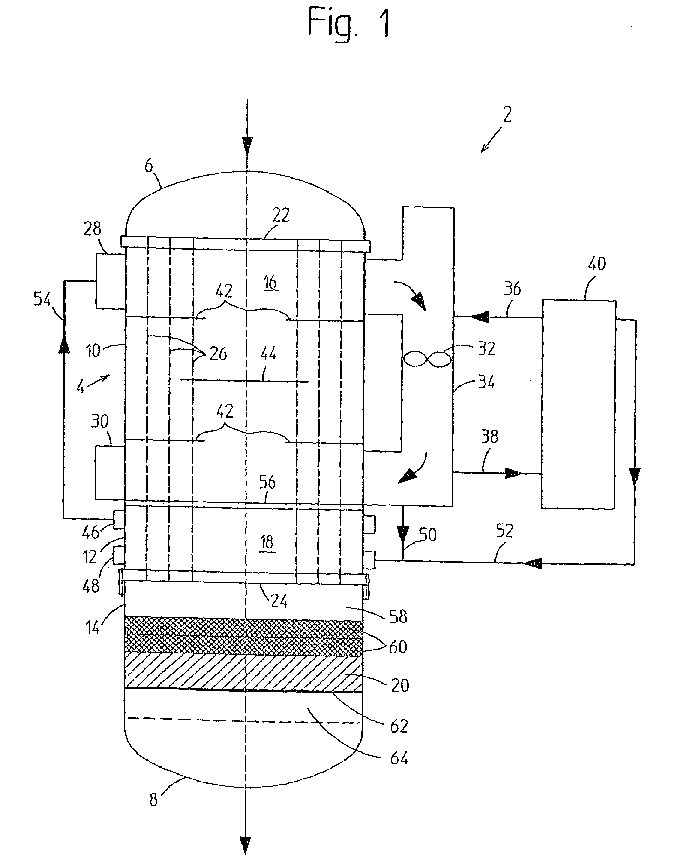

Die in Fig. 1 gezeigte Reaktoranordnung 2 besitzt ein im ganzen zylindrisches Gehäuse 4 mit einer üblichen Gaseintrittshaube 6 und Gasaustrittshaube 8. Der Mantel des Gehäuses 4 ist in einzelne Abschnitte 10, 12 und 14 unterteilt entsprechend einem Hauptreaktor in Gestalt eines üblichen Röhrenreaktors 16, einer Kühlstufe 18 und einem Nachreaktor 20 in Gestalt eines ungekühlten Festbettreaktors. Die Mantelabschnitte 10 und 12 sind zusammenhängend, d.h. durchlaufend, ausgebildet und enden an einem oberseitigen Rohrboden 22 bzw. einem unterseitigen Rohrboden 24, wozwischen sich in bei Röhrenreaktoren gewohnter Weise ein von einem Kühlmittel in Gestalt eines Salzbades umströmtes Rohrbündel 26 erstreckt. Die Gaseintrittshaube 6 ist auf nicht näher gezeigte Weise an den Rohrboden 22 angeflanscht, ebenso wie der Mantelabschnitt 14 mit der Gasaustrittshaube 8 an den Rohrboden 24 angeflanscht ist. In der Nähe seiner beiden Enden befinden sich an dem Mantelabschnitt 10 in wiederum bei Röhrenreaktoren üblicher Weise Ringkanäle 28 und 30, durch die das Kühlmittel aus dem Reaktor abgezogen bzw. nach Passieren einer Pumpe 32 dem Reaktor wieder zugeführt wird. Vor und nach der Pumpe 32 ist an das betreffende Pumpengehäuse 34 über Leitungen 36 und 38 ein Kühler 40 angeschlossen, durch den ein regelbarer Teilstrom des durch die Pumpe 32 umgewälzten Kühlmittels hindurchgeleitet wird. Im Inneren des Reaktors 16 können sich, wie gezeigt, in wiederum üblicher Weise Leitbleche 42 und 44 befinden, um dem Kühlmittel im Bereich des Rohrbündels 26 eine radiale Strömungskomponente zu erteilen. Die Leitbleche 42 und 44 können, wie gezeigt, aus an sich bekannten Ring- und Kreisscheiben bestehen und, davon abgesehen, - ggf. variierende - Durchtrittsöffnungen für das Kühlmittel um die Rohre herum wie auch zwischen den Rohren des Rohrbündels 26 aufweisen.

Zweckmäßigerweise beträgt die Temperaturdifferenz über das Rohrbündel 26 hinweg in einer Ebene weniger als 15°C und diejenige zwischen Reaktorein- und -austritt (Ringkanal 30 bzw. 28) weniger als 30°C. Axial gesehen kann das Kühlmittel durch den Röhrenreaktor 16 im Gleichstrom wie im Gegenstrom in bezug auf das Reaktionsgas hindurchtreten. Prinzipiell kann das Reaktionsgas auch abweichend von Fig. 1 von unten nach oben durch die Reaktoranordnng 2 hindurchtreten, wobei dann freilich die gesamte Anordnung gewissermaßen auf den Kopf zu stellen ist. Ferner kann der Röhrenreaktor 16 bereits für sich mehrere Reaktionszonen mit mehr oder weniger voneinander getrennten Kühlmittelkreisläufen und ggf. auch unterschiedlichen Katalysatoren gemäß DE 2 201 528 A aufweisen.

Ungeachtet der Unterbringung von Röhrenreaktor 16, Kühlstufe 18 und Nachreaktor 20 in ein- und demselben Gehäuse gestattet es der Aufbau etwa nach Fig. 1, diese Elemente leicht voneinander zu trennen. Hinsichtlich des Katalysatoraustauschs kann es zweckmäßig sein, den Katalysator des Nachreaktors 20 zu einem anderen Zeitpunkt zu erneuern als denjenigen des Röhrenreaktors 16. Dabei ist es auch denkbar, den einen oder anderen Katalysator nur teilweise zu ersetzen, den Katalysator des Röhrenreaktors 16 anschließend im Nachreaktor 20 einzusetzen oder umgekehrt, und dergl. mehr.

Abweichend von Fig. 1 kann der Röhrenreaktor 16 im Bedarfsfall prinzipiell von solcher Art sein, wie in DE 2 201 528 A und/oder DE 34 09 159 A angegeben, d.h. mit mehreren aufeinanderfolgenden, voneinander ganz oder teilweise getrennten Reaktionszonen oder mit mehreren Pumpen an ein- und denselben Ringkanälen, und/oder er kann in seinem Inneren einen oder mehrere Bypässe für das Kühlmittel nach WO 90/06807 aufweisen und dergl. mehr.

Im Beispiel der Fig. 1 tritt durch die Kühlstufe 18 in Gestalt eines Röhrenkühlers über diesem eigene Ringkanäle 46 und 48 ein wiederum regelbarer Teilstrom des gleichen Kühlmittels hindurch, wie es auch in dem Röhrenreaktor 16 Anwendung findet, wobei dieser Teilstrom aus zwei Strömen mischbar ist, die einerseits über eine Leitung 50 vom Eintritt in den Röhrenreaktor 16, andererseits über eine Leitung 52 vom Austritt des Kühlers 40 stammen. Der Rückfluß erfolgt über eine Leitung 54 von dem Ringkanal 46 zu dem Ringkanal 28.

Die durch die Kühlstufe 18 hindurch umgewälzte Wärmeträgermenge muß den Wert Mₘᵢₙ = Q/(c*Δt) überschreiten mit
- Q[W] =: von der Kühlstufe 18 abzuführende Wärmemenge,
- c*[J/kg °C] =: spezifische Wärmekapazität und
- Δt[°C] =: Temperaturdifferenz zwischen Kühlmittelaustrittstemperatur am Reaktor und Prozeßgastemperatur am Austritt der Kühlstufe 18

Indessen kann und soll sich die von der Kühlstufe 18 herbeigeführte Temperaturabsenkung des Reaktionsgases auf maximal 120°C beschränken.

Erforderlichenfalls kann die Leitung 54 auch zu einer anderen Stelle des Kühlmittelkreislaufs führen, etwa einer solchen saugseitig unmittelbar vor der Pumpe 32, um so ein ausreichend hohes Druckgefälle für die Kühlstufe 18 zu erhalten. Darüber hinaus kann die Kühlstufe 18 selbstverständlich auch mit einem eigenen Kühlsystem betrieben werden.

Solange in dem Röhrenreaktor 16 und der Kühlstufe 18 das gleiche Kühlmittel Verwendung findet, genügt es, die Kühlstufe 18 von dem Röhrenreaktor 16 lediglich durch eine - nicht sorgfältig abdichtende - Trennplatte 56 zu trennen. Gewünschtenfalls kann jedoch durch Einwalzen der Rohre, wie in DE 2 201 528 A angesprochen, eine sehr weitgehende wenn nicht sogar vollkommene Abdichtung erreicht werden, entsprechend einer Leckage von jedenfalls < 1 Liter pro Stunde und Rohr.

Davon unabhängig können die Rohre des Rohrbündels 26 auch noch innerhalb der Kühlstufe 18 mit Katalysator, ebenso aber auch mit einem Inertmaterial gefüllt sein, oder es können dort Drahtspiralen oder dergl. eingebaut sein, um die Turbulenz des Gases und damit den Wärmeübertritt zu verbessern.

Dem Nachreaktor 20 ist eine Gassammelzone 58 gefolgt von einem Mischer 60 vorgeschaltet, der aus einer Schüttung eines Inertmaterials, aus Metallstreifen oder, wie gezeigt, aus zwei Lagen eines Drahtgeflechts, aus profilierten Lochplatten oder dergl. bestehen kann. Der Nachreaktor 20 ist ein Festbettreaktor aus einer Katalysatorschüttung, die auf einer gasdurchlässigen Tragplatte 62 aufliegt. An den Nachreaktor 20 kann sich, wie gestrichelt angedeutet, eine Nachkühlstufe 64 anschließen, um das aus dem Nachreaktor austretende Reaktionsgas auf eine für einen PSA-Abscheider verträgliche Temperatur abzukühlen. Die Nachkühlstufe 64 kann in ähnlicher Weise gestaltet sein wie die Kühlstufe 18 oder in irgendeiner anderen geläufigen Weise.

Bemerkenswert bei der so weit beschriebenen Reaktoranordnung 2 ist, daß sich die Gassammelzone 58, vor allem bei Verwendung eines nachfolgenden Mischers, wie des gezeigten Mischers 60, fast nach Belieben verkleinern läßt. Da dem aus dem Röhrenreaktor 16 austretenden Gas innerhalb der Kühlstufe 18 nur das Rohrvolumen zur Verfügung steht, das sich zudem noch, etwa durch eine Füllung, verkleinern läßt, verringert sich dementsprechend das Explosionsrisiko selbst bei hoher Beladung. Bekanntermaßen ist das Explosionsrisiko bei bestimmten Reaktionsprodukten und bestimmter Beladung nämlich, abgesehen von der Temperatur, von dem den Reaktionsgasen zur Verfügung stehenden Volumen und von der Verweildauer der Reaktionsgase darin abhängig. Sollte es dennoch einmal zu einer Explosion kommen, so ist dieser nur ein geringes Gasvolumen unterworfen, wodurch sich der Druckanstieg in beherrschbaren Grenzen hält.

Aus den vorgenannten Gründen - Verringerung des Explosionsrisikos; Verringerung des Explosionsdrucks - wird bei dem nach der Erfindung zum Einsatz kommenden Röhrenreaktor 16 der Gaseintrittsbereich (Gaseintrittshaube; gaseintrittsseitiger Rohrboden) zweckmäßigerweise in der Weise gestaltet, wie in DE 198 06 810 A1 angegeben.

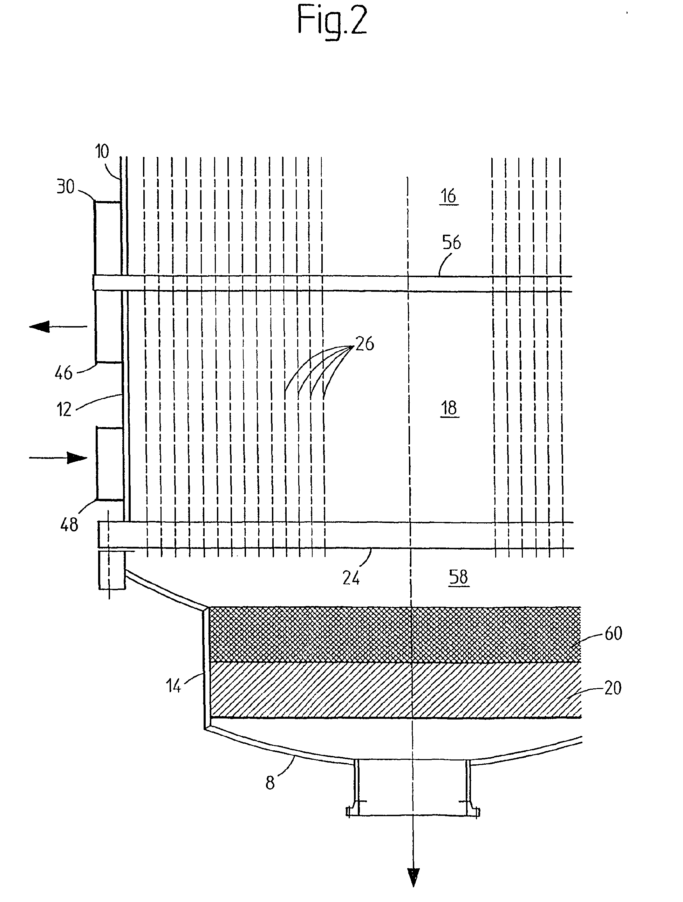

Die in Fig. 2 (ausschnittsweise) gezeigte Reaktoranordnung gleicht im Prinzip derjenigen aus Fig. 1. Entsprechend sind gleichartige Elemente mit den gleichen Bezugszahlen bezeichnet.

Zu erkennen ist das untere Ende des Röhrenreaktors 16 mit der darunterliegenden Kühlstufe 18, voneinander getrennt durch die Trennplatte 56, sowie der unterseitig darauffolgende Nachreaktor 20 mit vorausgehendem Mischer 60 und davorliegender Gassammelzone 58. Gegenüber Fig. 1 ist indessen zur Erhöhung der Strömungsgeschwindigkeit in dem Nachreaktor 20 dessen Durchmesser und damit sein Querschnitt verringert.

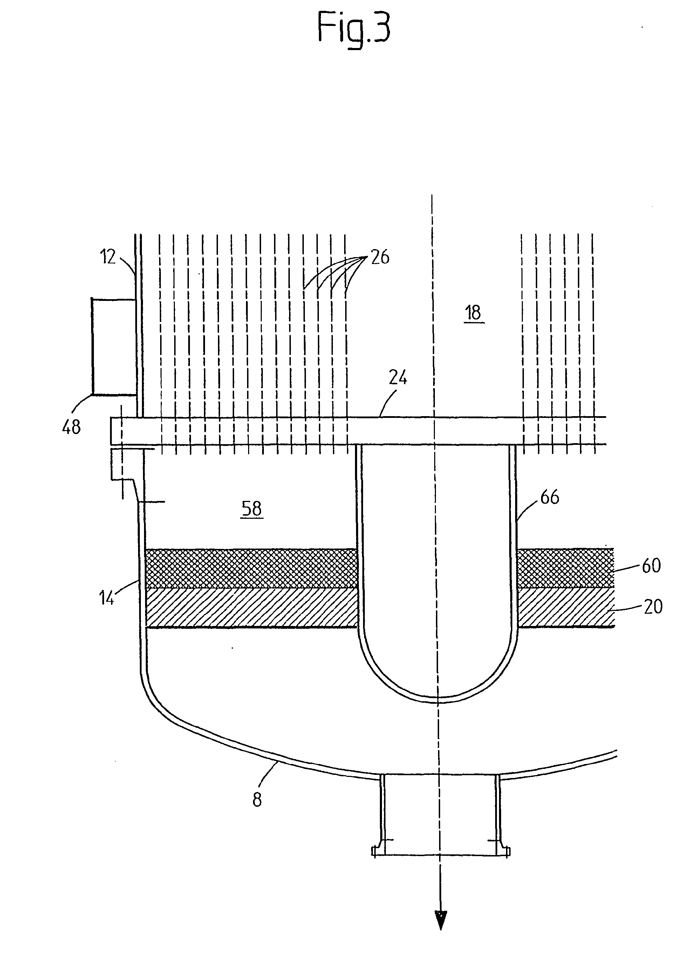

Gemäß Fig. 3 ist eine ähnliche Querschnittsverringerung des Nachreaktors 20 bei sonst ähnlicher Ausbildung wie nach Fig. 1 durch einen zentralen Verdrängerkörper 66 erreicht.

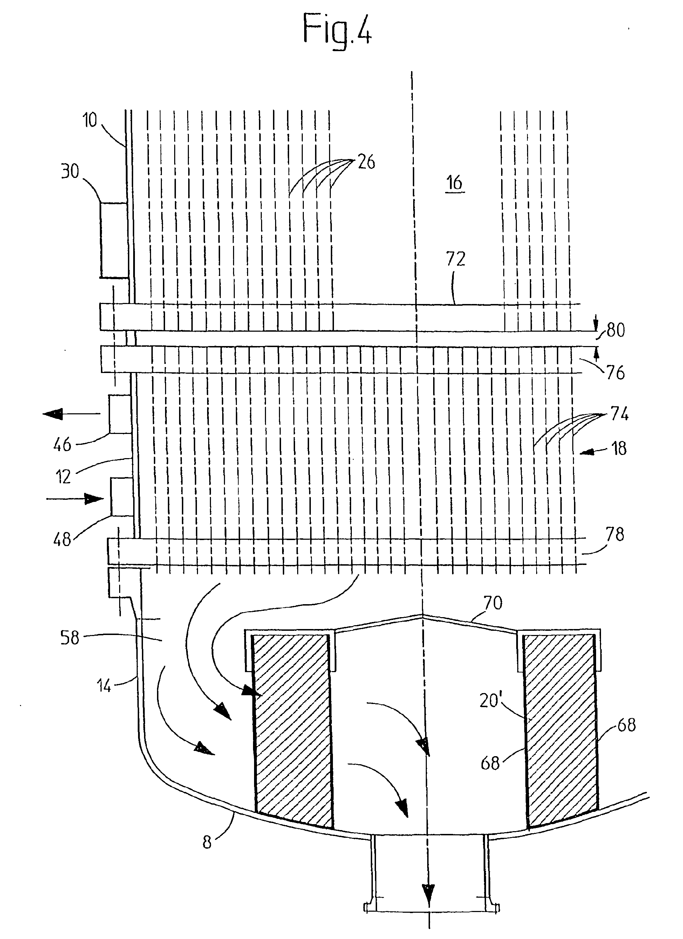

In Fig. 4 ist ein Nachreaktor 20' hohlzylindrischer Gestalt gezeigt, wie er gleichfalls auf die erfindungsgemäße Weise Anwendung finden kann. In diesem Fall wird der Katalysator von einem Drahtkorb 68 aufgenommen. Eine Abdeckung 70 verhindert einen unmittelbaren Austritt des Reaktionsgases unter Umgehung des Nachreaktors 20'.

In weiterer Abweichung von den vorausgehenden Figuren zeigt Fig. 4 beispielhaft eine vollkommene Trennung der Kühlstufe 18 von dem Röhrenreaktor 16, wozu der Röhrenreaktor 16 mit einem eigenen Rohrboden, 72, abschließt und die Kühlstufe 18 ein eigenes Rohrbündel 74 aufweist. Dementsprechend besitzt die Kühlstufe 18 zwei eigene Rohrböden, 76 und 78, von denen der Rohrboden 76 dem Rohrboden 72 des Röhrenreaktors 16 mit geringem Abstand 80 gegenüberliegt. Bei genügendem Abstand 80 brauchen die Rohre des Rohrbündels 74 nicht, wie gezeigt, mit denen des Rohrbündels 26 zu fluchten. Auch kann die Zahl der Rohre der Kühlstufe 18 von derjenigen des Röhrenreaktors 16 verschieden sein, ebenso wie die von den Rohren eingenommene Querschnittsfläche.

Dazu noch kann der den Abstand 80 bildende Raum einen Mischer aufnehmen ähnlich dem Mischer 60 aus den Figuren 1 - 3, um das aus dem Röhrenreaktor 16 austretende Prozeßgas vor Eintritt in das Rohrbündel 74 der Kühlstufe 18 zu homogenisieren.

Auf jeden Fall empfiehlt es sich, den betreffenden Raum, wie im übrigen auch den Raum 58 zwischen Kühlstufe 18 und Nachreaktor 20 bzw. 20', oder zumindest die darin vorhandenen freien Volumina möglichst klein zu machen, um das Explosionsrisiko und, im Falle einer dennoch eintretenden Explosion, den Druckanstieg gering zu halten.

Es versteht sich, daß bei getrennter Ausführung der Kühlstufe 18, etwa nach Fig. 4, das Kühlmittel innerhalb der Kühlstufe 18 nicht das gleiche zu sein braucht wie dasjenige im Röhrenreaktor 16. So kommt als Kühlmittel für die Kühlstufe 18 beispielsweise auch Druckwasser in Betracht.

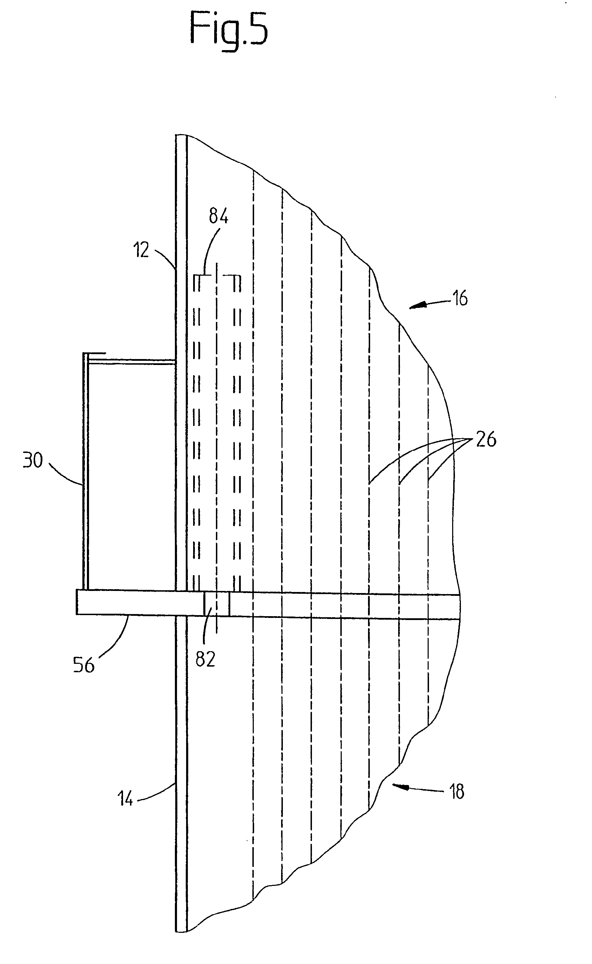

Fig. 5 zeigt - sehr schematisch - wiederum einen demjenigen aus Fig. 1 vergleichbaren Aufbau, indem hiernach ein Rohrbündel 26 dem Röhrenreaktor 16 und der Kühlstufe 18 gemeinsam ist und die beiden letzteren lediglich durch eine Trennplatte, 56, voneinander getrennt sind. Hier jedoch tritt an die Stelle eines Ringkanals, wie des Ringkanals 46, für den Kühlmittelaustritt aus der Kühlstufe 18 eine Anzahl Durchbrechungen in Gestalt kalibrierter Bohrungen 82 in der Trennplatte 56, durch die das Kühlmittel aus der Kühlstufe 18 anstatt über eine Leitung, wie die Leitung 54 aus Fig. 1, unmittelbar in den Röhrenreaktor 16 übertritt.

Zur Erhöhung des Druckgefälles für den Kühlmittelübertritt können sich, wie in Fig. 5 gestrichelt dargestellt, an die Bohrungen 82 mehr oder weniger weit zu dem Kühlmittelaustrittsende des Röhrenreaktors 16 hin führende Rohre 84 anschließen. Andererseits können funktionsgleiche Durchbrechungen wie die in Fig. 5 gezeigten Bohrungen 82 auch von um zumindest einen Teil der Rohre des Rohrbündels 26 herum bewußt ausgebildeten Ringspalten gebildet werden.

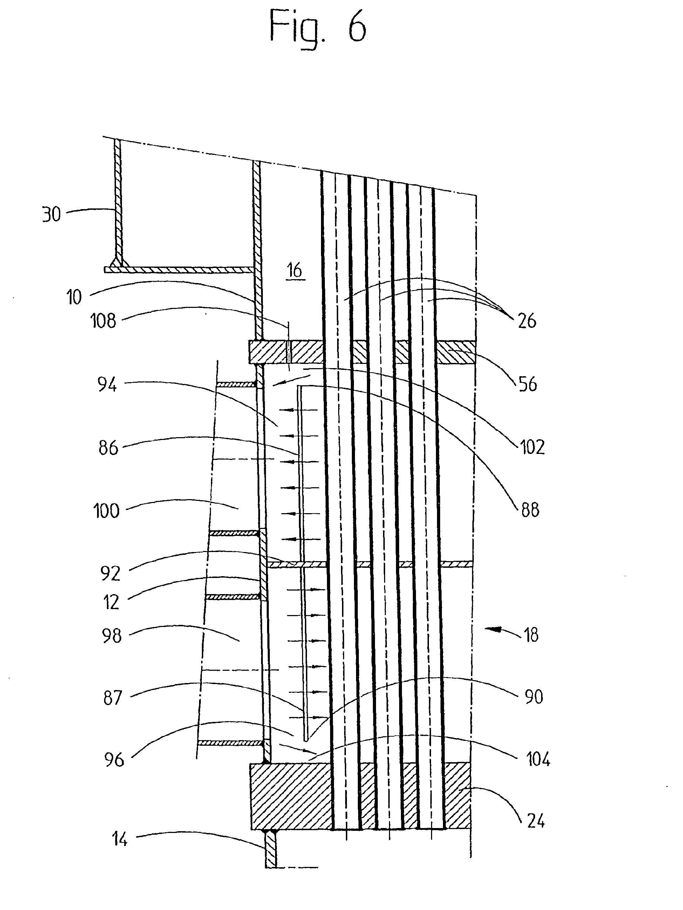

Gemäß Fig. 6 können bei einer Kühlstufe 18 mit Ringkanälen diese abweichend von den Ringkanälen 46 und 48 nach den Figuren 1 bis 4 auch in das Innere der Kühlzone, genauer gesagt des Mantelabschnitts 12, verlegt werden. Dazu befinden sich an der Peripherie außerhalb des Rohrbündels 26 gelochte zylindrische Dosierplatten 86 und 87, die mit ihren Rändern 88 und 90 einerseits nicht bis zu der Trennscheibe 56 bzw. dem Rohrboden 76, andererseits nicht bis zu dem Rohrboden 24 bzw. 78 reichen. Eine horizontale ringförmige Trennscheibe 92 teilt den außerhalb der Dosierplatten 86 und 87 liegenden Ringraum in zwei übereinanderliegende Abschnitte 94 und 96 auf, die mit einem Eintrittsstutzen 98 bzw. einem Austrittsstutzen 100 für das Kühlmittel in Verbindung stehen. Dabei können die Ringspalte 102 und 104 über bzw. unter den Dosierplatten 86 und 87 wie auch deren Lochgröße und/oder Lochabstand über den Umfang hinweg so variieren, daß der Zu- bzw. Austritt des Kühlmittels über den Umfang der Dosierplatten 86 hinweg im wesentlichen konstant ist. Unter Umständen können auch die Ringspalte 102 und 104 oder aber die Löcher der Dosierplatten 86 und 87 entfallen. Im Inneren der Kühlstufe 18 sind analog dem Röhrenreaktor 16 Umlenkbleche (nicht dargestellt) angeordnet.

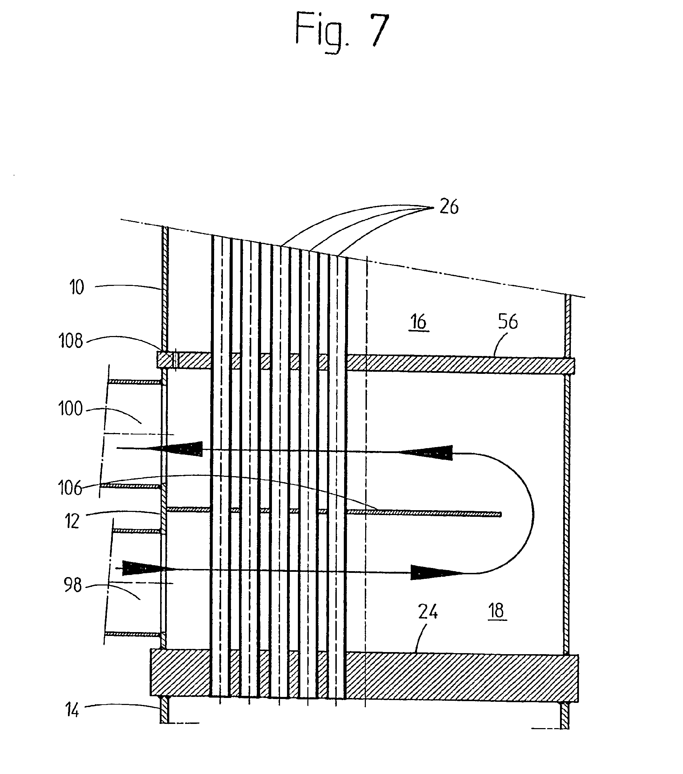

Fig. 7 zeigt, wie in einer Kühlstufe 18 wieder anderer Bauart durch seitliche Eintritts- und Austrittsstutzen 98 bzw. 100 ähnlich denjenigen nach Fig. 6 in Verbindung mit einem horizontalen Leitblech 106 eine U-förmige Querströmung des Kühlmittels erreicht werden kann. Sodann ist in Fig. 7 - lediglich beispielhaft - noch ein Entgasungskanal 108 zur Entgasung des Kühlmittels aus dem Inneren der Kühlstufe 18 zu erkennen, wie er üblicherweise auch bei Röhrenreaktoren Anwendung findet.

Es versteht sich, daß die vorausgehend beschriebenen Einzelaggregate und deren Teile mancherlei Abwandlungen erfahren können. So etwa kann der Hauptreaktor 16 ein Mehrzonenreaktor sein, oder es können die Rohre einer Kühlstufe 18 in Gestalt eines Röhrenkühlers strömungsbeeinflussende Einbauten und/oder eine Inertmaterialfüllung enthalten.

## Patentansprüche

1. Reaktoranordnung (2) für die Durchführung katalytischer Gasphasenreaktionen, insbesondere zur Gewinnung von Phthalsäureanhydrid aus o-Xylol und/oder Naphthalin, mit einem eine Katalysatorfüllung aufweisenden Hauptreaktor (16), der eine Kühleinrichtung (32, 34, 40) aufweist, und einem diesem nachgeschalteten Nachreaktor (20; 20'), wobei sich Hauptreaktor (16) und Nachreaktor (20; 20') in ein- und demselben Gehäuse (4) befinden, **dadurch gekennzeichnet, daß**
der Hauptreaktor (16) ein flüssigkeitsgekühlter Röhrenreaktor ist und zwischen Röhrenreaktor (16) und Nachreaktor (20; 20') eine Kühlstufe (18) geschaltet ist, die von einem Röhrenkühler gebildet wird und sich ebenfalls in dem Gehäuse (4) befindet.

2. Reaktoranordnung (2) nach Anspruch 1, **dadurch gekennzeichnet, daß** zwischen Hauptreaktor (16) und Nachreaktor (20; 20') ein Mischer (60) zwischengeschaltet ist.

3. Reaktoranordnung (2) nach Anspruch 1 oder 2, **dadurch gekennzeichnet, daß** die Kühlstufe (18) in einem Nebenschluß des Kühlmittelkreises für den Hauptreaktors (16) liegt.

4. Reaktoranordnung (2) nach Anspruch 3, **dadurch gekennzeichnet, daß** der Teilstrom für die Kühlstufe (18) separat regelbar ist.

5. Reaktoranordnung (2) nach einem der vorhergehenden Ansprüche, **dadurch gekennzeichnet, daß** die Rohre des Röhrenkühlers unmittelbare Fortsetzungen der Reaktionsrohre des Hauptreaktors (16) sind.

6. Reaktoranordnung (2) nach Anspruch 5 in Verbindung mit Anspruch 3, **dadurch gekennzeichnet, daß** die Kühlstufe (18) von dem Hauptreaktor (16) lediglich durch eine Trennplatte (56) getrennt ist, während sich ein Rohrboden (24) erst am Gasaustritt der Kühlstufe befindet.

7. Reaktoranordnung (2) nach Anspruch 3 oder 6, **dadurch gekennzeichnet, daß** der betreffende Teilstrom des Kühlmittels über mindestens eine Durchbrechung (82) zwischen Hauptreaktor (16) und Kühlstufe (18) im Inneren des Reaktorgehäuses (4) zustande kommt.

8. Reaktoranordnung (2) nach Anspruch 7, **dadurch gekennzeichnet, daß** sich an die Durchbrechung (82) zur Vergrößerung des Druckgefälles ein sich zum Wärmeträgereintrittsende des Hauptreaktors (16) hin erstreckendes Rohr (84) anschließt.

9. Reaktoranordnung (2) nach Anspruch 7 in Verbindung mit Anspruch 6, **dadurch gekennzeichnet, daß** eine Mehrzahl solcher Durchbrechungen von um zumindest einen Teil der Rohre herum ausgebildeten Ringspalten der Trennplatte (56) gebildet werden.

10. Reaktoranordnung (2) nach einem der vorhergehenden Ansprüche, **dadurch gekennzeichnet, daß** die Kühlstufe (18) Ringkanäle (46, 48) für die periphere Zu- bzw. Abführung des durch die Kühlstufe hindurchgeleiteten Kühlmittels aufweist.

11. Reaktoranordnung (2) nach Anspruch 10, **dadurch gekennzeichnet, daß** die betreffenden Ringkanäle sich im Inneren des Mantels der Kühlstufe (18) befinden.

12. Reaktoranordnung (2) nach einem der vorhergehenden Ansprüche, **dadurch gekennzeichnet, daß** die Kühlstufe (18) mindestens ein eine Querströmung des durch sie hindurchgeleiteten Kühlmittels herbeiführendes Leitblech (106) enthält.

13. Reaktoranordnung (2) nach einem der vorhergehenden Ansprüche, **dadurch gekennzeichnet, daß** die Rohre des Röhrenkühlers strömungsbeeinflussende Einbauten und/oder eine Inertmaterialfüllung enthalten.

14. Reaktoranordnung (2) nach einem der vorhergehenden Ansprüche, **dadurch gekennzeichnet, daß** die Kühlstufe (18) entsprechend ausgelegt und/oder geregelt ist, eine Temperaturabsenkung des Reaktionsgases um maximal 120°C zu bewirken.

15. Reaktoranordnung (2) nach einem der vorhergehenden Ansprüche, **dadurch gekennzeichnet, daß** zwischen Hauptreaktor (16) und Kühlstufe (18) ein Mischer zwischengeschaltet ist.

16. Reaktoranordnung (2) nach einem der vorhergehenden Ansprüche, **dadurch gekennzeichnet, daß** der Hauptreaktor (16) ein Mehrzonenreaktor ist.

17. Reaktoranordnung (2) nach einem der vorhergehenden Ansprüche, **dadurch gekennzeichnet, daß** der Nachreaktor (20; 20') ein ungekühlter Festbettreaktor ist.

## Claims

1. Reactor arrangement (2) for carrying out catalytic gas phase reactions, in particular for obtaining phthalic anhydride from o-xylene and/or naphthalene, with a main reactor (16) which has a catalyst filling and has a cooling device (32, 34, 40), and with a secondary reactor (20; 20') following the said main reactor, the main reactor (16) and secondary reactor (20; 20') being located in one and the same housing (4), **characterized in that** the main reactor (16) is a liquid-cooled tubular reactor, and, between the tubular reactor (16) and secondary reactor (20; 20'), a cooling stage (18) is inserted, which is formed by a tubular cooler and is likewise located in the housing (4).

2. Reactor arrangement (2) according to Claim 1, **characterized in that** a mixer (60) is interposed between the main reactor (16) and secondary reactor (20; 20').

3. Reactor arrangement (2) according to Claim 1 or 2, **characterized in that** the cooling stage (18) lies in a shunt of the coolant circuit for the main reactor (16).

4. Reactor arrangement (2) according to Claim 3, **characterized in that** the part-stream for the cooling stage (18) can be regulated separately.

5. Reactor arrangement (2) according to one of the preceding claims, **characterized in that** the tubes of the tubular cooler are direct continuations of the reaction tubes of the main reactor (16).

6. Reactor arrangement (2) according to Claim 5 in conjunction with Claim 3, **characterized in that** the cooling stage (18) is separated from the main reactor (16) solely by a separating plate (56), whilst a tube bottom (24) is located only at the gas outlet of the cooling stage.

7. Reactor arrangement (2) according to Claim 3 or 6, **characterized in that** the respective part-stream of the coolant occurs inside the reactor housing (4) via at least one perforation (82) between the main reactor (16) and cooling stage (18).

8. Reactor arrangement (2) according to Claim 7, **characterized in that** a tube (84) extending to the heat transfer medium inlet end of the main reactor (16) adjoins the perforation (82) in order to increase the pressure drop.

9. Reactor arrangement (2) according to Claim 7 in conjunction with Claim 6, **characterized in that** a plurality of such perforations are formed by annular gaps of the separating plate (56) which are formed around at least some of the tubes.

10. Reactor arrangement (2) according to one of the preceding claims, **characterized in that** the cooling stage (18) has annular ducts (46, 48) for the peripheral supply and discharge of the coolant conducted through the cooling stage.

11. Reactor arrangement (2) according to Claim 10, **characterized in that** the respective annular ducts are located inside the casing of the cooling stage (18).

12. Reactor arrangement (2) according to one of the preceding claims, **characterized in that** the cooling stage (18) contains at least one guide plate (106) which brings about a transverse flow of the coolant conducted through the said cooling stage.

13. Reactor arrangement (2) according to one of the preceding claims, **characterized in that** the tubes of the tubular cooler contain flow-influencing fittings and/or an inert-material filling.

14. Reactor arrangement (2) according to one of the preceding claims, **characterized in that** the cooling stage (18) is appropriately designed and/or regulated in order to bring about a drop in temperature of the reaction gas by the amount of a maximum of 120°C.

15. Reactor arrangement (2) according to one of the preceding claims, **characterized in that** a mixer is interposed between the main reactor (16) and cooling stage (18).

16. Reactor arrangement (2) according to one of the preceding claims, **characterized in that** the main reactor (16) is a multi-zone reactor.

17. Reactor arrangement (2) according to one of the preceding claims, **characterized in that** the secondary reactor (20; 20') is an uncooled fixed-bed reactor.

## Revendications

1. Agencement de réacteur (2) pour l'exécution de réactions catalytiques en phase gazeuse, en particulier pour la récupération d'anhydride d'acide phtalique à partir de o-xylol et/ou de naphtaline, comprenant un réacteur principal (16) qui présente le remplissage de catalyseur et qui présente un dispositif de refroidissement (32, 34, 30), et un réacteur secondaire (20 ; 20') branché à la suite, dans lequel le réacteur principal (16) et le réacteur secondaire (20 ; 20') se trouvent dans un seul et même boîtier (4), **caractérisé en ce que** le réacteur principal (16) est un réacteur à tubes refroidi par liquide, et entre le réacteur à tubes (16) et le réacteur secondaire (20 ; 20') est branché un étage de refroidissement (18) qui est formé par un refroidisseur à tubes et qui se trouve également dans le boîtier (4).

2. Agencement de réacteur (2) selon la revendication 1, **caractérisé en ce qu'**un mélangeur (60) est interposé entre le réacteur principal (16) et le réacteur secondaire (20 ; 20').

3. Agencement de réacteur (2) selon la revendication 1 ou 2, **caractérisé en ce que** l'étage de refroidissement (18) se trouve dans une dérivation du circuit de réfrigérant pour le réacteur principal (16).

4. Agencement de réacteur (2) selon la revendication 3, **caractérisé en ce que** le courant partiel pour l'étage de refroidissement (18) est susceptible d'être régulé séparément.

5. Agencement de réacteur (2) selon l'une des revendications précédentes, **caractérisé en ce que** les tubes du refroidisseur à tubes sont des prolongements immédiats des tubes de réactions du réacteur principal (16).

6. Agencement de réacteur (2) selon la revendication 5 en dépendance de la revendication 3, **caractérisé en ce que** l'étage de refroidissement (18) est séparé du réacteur principal (16) simplement par une plaque de séparation (56), tandis qu'un fond tubulaire (24) se trouve uniquement à la sortie des gaz de l'étage de refroidissement.

7. Agencement de réacteur (2) selon la revendication 3 ou 6, **caractérisé en ce que** le courant partiel concerné du réfrigérant se produit à l'intérieur du boîtier de réacteur (4) via au moins une traversée (82) entre le réacteur principal (16) et l'étage de refroidissement (18).

8. Agencement de réacteur (2) selon la revendication 7, **caractérisé en ce que**, pour augmenter le gradient de pression, un tube (84) qui s'étend vers l'extrémité d'entrée de l'agent caloporteur dans le réacteur principal (16) se raccorde à la traversée (82).

9. Agencement de réacteur (2) selon la revendication 7 en dépendance de la revendication 6, **caractérisé en ce qu'**une pluralité de telles traversées sont formées par des fentes annulaires, ménagées autour d'au moins une partie des tubes, de la plaque de séparation (56).

10. Agencement de réacteur (2) selon l'une des revendications précédentes, **caractérisé en ce que** l'étage de refroidissement (18) comprend des canaux annulaires (46, 48) pour l'admission ou l'évacuation périphérique du réfrigérant mené à travers l'étage de refroidissement.

11. Agencement de réacteur (2) selon la revendication 10, **caractérisé en ce que** les canaux annulaires concernés se trouvent à l'intérieur de l'enveloppe de l'étage de refroidissement (18).

12. Agencement de réacteur (2) selon l'une des revendications précédentes, **caractérisé en ce que** l'étage de refroidissement (18) contient au moins une tôle directrice (106) qui provoque un écoulement transversal du réfrigérant qui le traverse.

13. Agencement de réacteur (2) selon l'une des revendications précédentes, **caractérisé en ce que** les tubes du refroidisseur à tubes contiennent des inserts et/ou un remplissage de matériau inerte qui influence(nt) l'écoulement.

14. Agencement de réacteur (2) selon l'une des revendications précédentes, **caractérisé en ce que** l'étage de refroidissement (18) est conçu et/ou régulé de manière à provoquer une chute de température maximum de 120 °C dans le gaz de réaction.

15. Agencement de réacteur (2) selon l'une des revendications précédentes, **caractérisé en ce qu'**un mélangeur est interposé entre le réacteur principal (16) et l'étage de refroidissement (18).

16. Agencement de réacteur (2) selon l'une des revendications précédentes, **caractérisé en ce que** le réacteur principal (16) est un réacteur à zones multiples.

17. Agencement de réacteur (2) selon l'une des revendications précédentes, **caractérisé en ce que** le réacteur secondaire (20 ; 20') est un réacteur à lit solide non refroidi.
